# EUROPEAN PATENT SPECIFICATION

(11) **EP 4 054 691 B1**
(45) Date of publication and mention of the grant of the patent: **12.03.2025**
(21) Application number: 20799671.1
(22) Date of filing: 28.10.2020
(51) Int. Cl.: A61M 25/01, A61B 17/34, A61M 25/00, A61N 1/372, A61N 1/05, A61N 1/375, A61B 17/00

(54) **CATHETER TUBE FOR A STEERABLE CATHETER FOR IMPLANTING AN IMPLANTABLE MEDICAL DEVICE**
KATHETERSCHLAUCH FÜR EINEN STEUERBAREN KATHETER ZUM IMPLANTIEREN EINER IMPLANTIERBAREN MEDIZINISCHEN VORRICHTUNG
TUYAU DE CATHÉTER POUR UN CATHÉTER ORIENTABLE POUR L'IMPLANTATION D'UN DISPOSITIF MÉDICAL

(30) Priority: 05.11.2019 EP 19207026
(43) Date of publication of application: 14.09.2022
(73) Proprietor: BIOTRONIK SE & Co. KG, 12359 Berlin (DE)
(72) Inventor: RICHTER, Jan Helge, 12355 Berlin (DE); ZEDLER, Stephan, 12309 Berlin (DE); JADWIZAK, Detmar, 15537 Erkner (DE); SCHLODDER, Karsten, 15517 Fürstenwalde (DE)
(74) Representative: Biotronik Corporate Services SE
(86) International application number: PCT/EP2020/080221
(87) International publication number: WO 2021/089374

(56) References cited:
- WO-A1-2019/161286
- US-A1- 2012 232 563
- US-A1- 2018 296 801
- US-A1- 2019 192 820
- US-A1- 2019 201 688

## Description

The invention relates to a catheter tube for a steerable catheter. The invention can be used in a method for implanting an implantable medical device by means of a steerable catheter comprising such a catheter tube.

Catheter tubes are used in many fields of medicine. Implantation catheters for cardiac pacemakers (for example for an implantable leadless pacemaker, ILP) or for sensors for measuring a pulmonary artery pressure shall be mentioned by way of example. Within the scope of the present application, the terms catheter or catheter tube shall be understood to mean that these also encompass sheaths, such as introducer sheaths for electrode leads for HIS bundle pacing.

Such catheter tubes usually comprise a tube wall, which surrounds one or more tube lumens. The tube wall can at least partially consist of a relatively flexible plastic material, such as silicone (SI), polyether block amide (PEBA, for example PEBAX), polyurethane (PU), polyamide (PA) or polyethylene (PE), so that the catheter tube can easily follow the course of the vessels of a patient. Depending on the application, a distal end of the catheter tube is, in general, to be guided to a particular location in the body of the patient, for example so as to place an implant there, carry out diagnostics, or carry out a treatment, such as a cryo treatment, a targeted drug delivery or ablation. A proximal end of the catheter tube can, for example, transition into a rigid catheter shaft, which remains outside the patient's body. For example, a physician can grab a grip at the catheter shaft so as to advance the catheter or retract it.

In some known catheters in particular a distal portion of the catheter tube exhibits a certain steerability. This means, for example, that an orientation of the distal end of the catheter tube can be changed in a controlled manner, for example so as to position an implant in a targeted manner at a desired implantation site, or so as to act on the tissue of the patient in another manner. The steering can take place, for example, by the actuation of a steering mechanism at a grip of the catheter shaft.

For example, WO 01/37920 A1 describes a steerable catheter comprising a shaft, which was preformed during production with a twist at least in regions. The pre-twisted portion of the shaft can be deformed by pulling at a pull wire, which is attached at the distal end of the shaft and extends through a lumen in the shaft, so that this portion of the shaft achieves a desired (for example helical) curvature.

The disadvantage of approaches of this type, however, is that the formed curve is comparatively soft since the catheter has to be relatively flexible in the region in question. As a result, only comparatively low forces can be transmitted via the distal catheter end. For example, it is only possible with limited force to laterally press the distal catheter end against a vessel wall. It is also almost impossible to transmit a pushing force by way of this concept since a considerable portion of the pressure that is introduced axially through the grip is compensated for by the soft distal catheter end. Moreover, such approaches only allow a relatively small central lumen and are thus not as suitable for an introducer sheath, for example.

US 2007/0093780 A1 discloses an intravascular treatment device in which multiple threads are attached in a corkscrew-like manner on the outside of a distal portion of a catheter, so as to rotationally engage surrounding arterial tissue.

Furthermore, a catheter which is deformable in a spiral-shaped manner by at least two control wires is known from US 2007/0270679 A1.

Further steerable catheters are known from US 2019/201688 A1, US 2018/296801 A1, US 2019/192820 A1, US 2012/232563 A1 and WO 2019/161286 A1.

It is the object of the present invention to propose an improved catheter tube for a steerable catheter, in particular with respect to dimensional stability and ease of use.

Proceeding from this, the present invention provides a catheter tube according to claim 1. Features of several exemplary embodiments are described in the dependent claims.

Due to the guide lumen for the pull element being embedded into a mesh inside the tube wall, high dimensional stability (with good reproducibility of the shape) of the catheter tube can be achieved. In this way, high pushing forces can be transmitted from the proximal portion into a distal end of the catheter tube. The catheter tube can thus additionally have a sufficiently rigid design, so as to allow, for example, secure guidance of an inner catheter or of an electrode lead.

The design according to the invention can additionally impart sufficient dimensional stability (in particular torsional stability) to the catheter tube so as to allow a relatively high lateral force to be exerted, into the distal end of the catheter tube, by a rotation of the grip or the shaft. In this way, it is possible, for example, to exert comparatively high pressing forces onto the tissue.

By embedding the pull element into the mesh, the catheter tube has comparatively high stability against kinking. In particular, it can be avoided that the pull element, for example in the form of a pull wire, wears through an outer wall of the tube when the catheter tube is kinked or bent.

Another advantage of the proposed design of the tube wall is that, in this way, also comparatively large central lumens can be implemented. In this way, it is also possible, for example, to use the catheter tube as an introducer sheath. Such a sheath can, for example, be designed as a slittable sheath for placing electrode leads, sensors, or pacemakers in locations in the body that are difficult to access, in particular in the heart.

A few embodiments of a catheter tube according to the first aspect of the invention are described hereafter:
It may be provided, for example, that the guide lumen extends across a circumferential angle of at least 30° in the tube wall. This means that, when looking at the catheter tube in a state in which it extends linearly along a main extension axis, the guide lumen, as seen in a projection along the main extension axis, extends across a region along the circumference of the tube wall which, based on a center of the catheter tube, spans an angle of at least 30°.

The circumferential angle can also be larger and be as large as 360°, for example. The latter applies, for example, in designs in which the guide lumen is guided at least once completely around the tube lumen, such as on a spiral-shaped or helical path.

In an advantageous embodiment, the guide lumen is delimited by a guide lumen tube. For example, the guide lumen tube can comprise Teflon material and, in particular, consist of Teflon. In this way, it can be ensured that the pull element, for transmitting a pulling force (or for relief), is able to slide within the tube wall and is not, for example, blocked by the mesh.

The catheter tube can furthermore be designed in such a way that the distal portion is deformable in three dimensions (or, as needed, also only two-dimensionally) by an actuation of the pull element. In a preferred embodiment, it may be sufficient for this purpose to actuate a single pull element. In other words, a controlled three-dimensional configuration of the catheter can be made possible using a single steering mechanism in the form of a pull wire. In this way, particularly easy handling of the steerable catheter can be made possible for the physician.

In other variant embodiments, however, it is also possible for multiple pull elements to be provided in the respective guide lumens of the described type. For example, through the use of multiple pull elements that are guided around the tube lumen in different manners, it is possible to combine multiple curve shapes with one another. It is thus possible to run additional pull elements inside or outside the mesh, for example so as to form one or more further curves, independently of a first curve, in a different direction. In this way, it is possible, for example, to additionally axially tilt a helical curve.

The catheter tube can, for example, be deformable by an actuation of the at least one pull element (in particular of a single pull element) in such a way that the distal portion of the catheter tube is bent with respect to a main extension axis of the proximal portion of the catheter tube. In this way, for example, a formation of an opposing curve can be made possible. This means that the distal end of the catheter tube, as a result of the controlled deformation, can point, for example, at least partially (that is, at least with one directional component) in a direction that is oriented opposite to the advancement direction of the catheter.

For example, the distal end of the catheter tube can be "bent" in the manner of a straw (two-dimensional deformation within a plane through the main extension axis). However, it is also possible to add other directional components to the deformation, so that overall a controlled three-dimensional deformation takes place.

In one embodiment, for example, the catheter tube can be deformed by an actuation of the at least one pull element (in particular of a single pull element) in such a way that the distal portion of the catheter tube describes at least a section of a spiral-shaped or helical path. The spiral-shaped or helical path can, for example, extend around a main extension axis of the proximal portion of the catheter tube.

For example, the catheter tube can be deformed by an actuation of the pull element in such a way that respective local extension axes of the distal portion, as seen in a projection along a main extension axis of the catheter tube (in particular along a main extension axis of the proximal portion), pass over an angle α of at least 30°. The passed-over angle, however, can also be larger and preferably be in the range of 30° to 270°.

In one embodiment, the catheter tube can furthermore be deformable by an actuation of the pull element in such a way that respective local extension axes of the distal portion, as seen in a projection along a main extension axis of the catheter tube (in particular along a main extension axis of the proximal portion), describe at least approximately a section of a circular arc having a radius in the range of 2 mm to 100 mm.

It is also within the scope of the invention that the catheter tube can comprise multiple tube segments having differing rigidities. The differing rigidities of the individual tube segments can, for example, be achieved by tube materials having different hardness levels and/or by a different number of intersecting points of the mesh in the respective tube segments. The rigidity can also be varied through the use of tube segments with and without mesh.

For example, as a result of such measures, the rigidity of the catheter tube may tend to decrease in the direction from the proximal portion toward the distal portion.

Preferably, in particular tube segments that can be deformed as intended are designed to be relatively soft in the distal portion of the catheter tube. In particular in the region of such deformable tube segments, the at least one pull element (including the guide lumen) can be at least partially guided around the tube lumen so as to effectuate the deformation during a pulling action. The proximal portion, in contrast, can have a relatively rigid design, and the at least one pull element can be guided in a substantially straight manner (that is, axially along the catheter tube) there.

The present invention can be used in an exemplary, non-claimed method for implanting an implantable medical device by means of a steerable catheter comprising such a catheter tube. The method comprises the following steps:
- inserting the catheter into the patient's body, and advancing the distal portion of the catheter tube into the vicinity of a desired implantation site;
- deforming the distal portion by actuating the pull element in such a way that a distal end of the catheter tube rests against the tissue;
- fixing the implantable medical device at the desired implantation site by means of the catheter; and
- removing the catheter from the patient's body.

For example, the catheter tube can be in a first, non-formed state, which can also be referred to as a relaxed or neutral state, during insertion and advancement. For example, it can be provided that no, or at the most a low, pulling force is exerted by means of the pull element (or the multiple pull elements) in the non-formed state.

As a result of an actuation of the at least one pull element, and in particular by the exertion of a pulling force at the pull element, the catheter tube can then be transferred into a second, formed state, in which the distal portion can, for example, be more rigid than in the first state. In the second state, the distal end of the catheter tube can, for example, be braced with a relatively high force against the tissue, such as against a vessel wall.

When the catheter tube is being removed from the patient's body, the catheter tube can, for example, be in the first, non-formed state again. In this way, the retraction of the catheter tube through the patient's vessels can be facilitated.

The implantable medical device is a cardiac pacemaker electrode lead, which is implanted in a location in the patient's heart suitable for HIS bundle pacing, using the exemplary method of the present disclosure.

The implantation can take place as follows, for example:
First, the subclavian vein is punctured. As an alternative, the cephalic vein can be dissected free and punctured (by piercing the vein with a cannula and syringe).

Thereafter, a Seldinger wire is inserted, and the piercing location is expanded by way of a dilator. Optionally, it is also possible to advance the sheath introducer.

Thereafter, the steerable catheter is introduced into the right atrium or, as an alternative, into the right ventricle. A cardiac pacemaker electrode lead suitable for HIS bundle pacing is advanced through the steerable catheter until the tip of the electrode lead ends up in the region of the distal end of the catheter.

The deformable distal portion of the catheter tube is then deformed by an actuation of the at least one pull element until the distal end of the catheter tube is located in the vicinity of the HIS bundle. An axial angle of the deflection of the distal end can be 30° to 270° in the process here, for example. A resulting radius is, for example, between 2 mm and 100 mm. By bracing the catheter against the tissue, secure positioning of the catheter with the electrode lead is ensured.

Using suitable methods, it can be determined whether the distal end of the electrode lead, together with the steerable 3D catheter, is situated sufficiently close to the HIS bundle. For this purpose, for example, an intracardiac ECG (IGM) or a sample stimulation by way of an external stimulator can be used.

Once the suitable position has been found, the electrode lead is advanced, with the inserted stylet, until the tip of the electrode lead is parietal. Thereafter, the fixation of the electrode lead is screwed into the cell tissue. A measurement is conducted as to whether successful pacing of the HIS bundle is possible in this position. If this is not the case, the fixation is loosened again. In this case, the last steps are repeated until a clinically meaningful position has been found.

The catheter tube is then relaxed and thus brought into the neutral state (in which, for example, no pulling force acts on the pull element) that was already used during the insertion of the catheter.

The catheter tube is finally slit open by way of a suitable "slitter tool," retracted and removed, so that the electrode lead is freed of the catheter and can be connected to the cardiac pacemaker.

In another example, the implantable medical device is a sensor, such as a pressure sensor, which is implanted into a pulmonary artery of the patient by means of the exemplary method of the present disclosure.

Still another example provides that the implantable medical device is an implantable leadless pacemaker (ILP), which is implanted into the heart of the patient by means of the exemplary method of the present disclosure.

Consistent with the two latter examples of the method, in particular the following steps can take place:
- puncturing the subclavian vein and expanding the piercing location by means of a dilator;
- placing an introducer sheath.
- The catheter is inserted through the introducer by way of the sensor, or by way of the ILP, and is pushed through the atrium into the ventricle. In the process, the three-dimensional steering of the catheter can be employed.
- The placement of the ILP preferably takes place septally or also in the apex. Other implantation sites, for example for sensors in the pulmonary artery, are likewise conceivable. For this purpose, additional aids, such as Seldinger wires, are used, if necessary.
- The deformable distal portion of the catheter tube is deformed by an actuation of the at least one pull element until the distal end of the catheter tube is located in the vicinity of the HIS bundle. An axial angle of the deflection of the distal end can be 30° to 270° in the process here, for example. A resulting radius is, for example, between 2 mm and 100 mm. By bracing the catheter against the tissue, secure positioning of the catheter with the electrode is ensured.
- The sensor or ILP situated at the distal end of the catheter tube is released with the aid of an inner catheter and is fixed in the tissue, or positioned in a vessel.
- It is determined by way of suitable methods whether the sensor, or the ILP, is correctly positioned. For this purpose, for example, an IGM or a sample stimulation can be carried out. If necessary, repositioning has to take place.
- The catheter tube is then relaxed and thus brought into the neutral state (in which, for example, no pulling force acts on the pull element) that was already used during the insertion of the catheter.
- The connection between the sensor/ILP and the catheter is released, and the catheter is removed.

In general, however, a catheter tube according to the invention can also be used in applications other than those described in greater detail above, such as for steering devices for cryo applications, as well as for targeted drug delivery, or for steering ablation or diagnostic catheters.

Further advantages and embodiments of the present invention shall be described hereafter with reference to the figures. In the drawings:
- FIG. 1: shows an exemplary embodiment of a catheter tube in a non-formed state;
- FIG. 2: shows a radial cross-section through the catheter tube of FIG. 1.
- FIG. 3: shows a longitudinal section through the catheter tube of FIG. 1;
- FIG. 4: shows the catheter tube of FIG. 1 in a formed state;
- FIG. 5: shows a formed distal portion of the catheter tube of FIG. 1 in a projection along a main extension axis of the catheter tube; and
- FIG. 6: shows a schematic block diagram including steps of a method for implanting an implantable medical device.

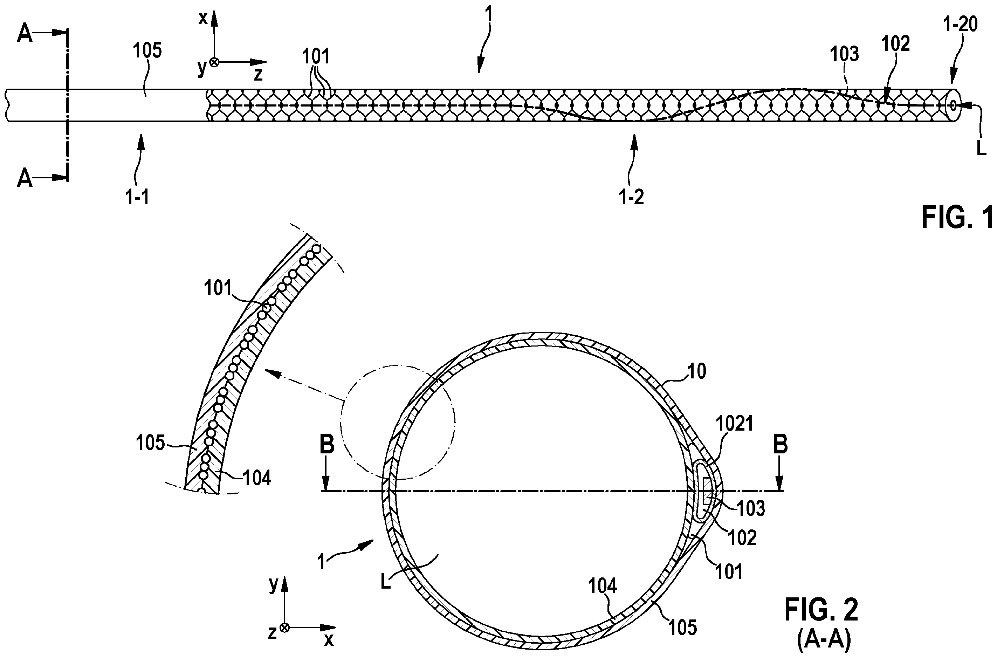

FIG. 1 schematically and by way of example shows a catheter tube 1 comprising a proximal portion 1-1 and a distal portion 1-2. The proximal portion 1-1 can, for example, transition into a rigid shaft (not shown). A grip can be arranged at the shaft, which a physician can use, for example, to handle the catheter. The distal portion 1-2, and in particular a distal end 1-20 of the catheter tube 1, is intended to be advanced within a patient's body to a location at which an implantation, a treatment or diagnostics is or are to be carried out.

The catheter tube 1 has a tube lumen L that is delimited by a tube wall 10. The tube wall 10 can, for example, be partially made of a flexible plastic material, such as silicone, which can also ensure sufficient tightness of the lumen. For example, a section of an outer layer 105 of the catheter 1 is shown in the proximal region in FIG. 1, which can be made of such a plastic material, for example.

Moreover, the tube wall 10 is reinforced by a mesh 101, such as a textile mesh, made of cotton or silk, for example, a wire mesh, preferably made of stainless steel or other metals, or a plastic mesh, made of polyamide (PA), polyurethane (PU), polyether block amide (PEBA, for example PEBAX), or polyether ether ketone (PEEK), for example. This is illustrated in FIG. 1 in particular in the distal portion 1-2 of the catheter tube 1. It shall be understood that the outer layer 105 can also extend in this portion, however it is not shown there for illustrative reasons so as to clearly show the mesh 101.

The tube wall 10 furthermore comprises a guide lumen 102 around which a mesh 101 is braided. A pull element 103, for example in the form of a pull wire, extends in the guide lumen 102, from the proximal portion 1-1 of the catheter tube 1 to the distal portion 1-2 of the catheter tube 1. The pull element 103 is connected in a tension-resistant manner to the tube wall 10 (and, if necessary, also to the mesh 101) in the distal portion 1-2.

The guide lumen 102 embedded into the mesh 101 guides the pull element 103 once completely around the tube lumen L in the distal portion 1-2 along a helical line. In other embodiments, it may be provided that the guide lumen 102 only describes a partial section of a helical path around the tube lumen L. In still other variant embodiments, the guide lumen 102 can even be helically guided multiple times around the tube lumen L.

For example, it may be provided that the guide lumen 102 extends across a circumferential angle of at least 30° in the tube wall 10. This means that, when looking at the catheter tube in a state in which it extends linearly along a main extension axis Z (as shown in FIG. 1), the guide lumen 102, as seen in a projection along the main extension axis Z, extends across a region along the circumference of the tube wall 10 which (based on a center of the catheter tube 1) spans an angle of at least 30°. The circumferential angle can also be larger and is, for example, 360° in the embodiment shown in FIG. 1. This is also the case, for example, in embodiments (which are not shown here) in which the guide lumen 102 is completely guided multiple times around the tube lumen L, as if on a spiral or helical path.

FIG. 2 schematically and by way of example shows a radial cross-section (along line A-A of FIG. 1) through the catheter tube 1. The cross-section A-A extends through a plane XY perpendicularly to the main extension direction Z of the catheter tube 1 of FIG. 1.

It is apparent based on the cross-section A-A that the tube lumen L is delimited by an inner layer 104 of the tube wall 10. Furthermore, the aforementioned outer layer 105 is apparent. The inner layer 104 and the outer layer 105 can, for example, be composed of one plastic material, or also of plastic materials that are different from one another, such as silicone (SI), polyurethane (PU), polyether block amide (PEBA, for example PEBAX), polyethylene (PE), or also polyamide (PA).

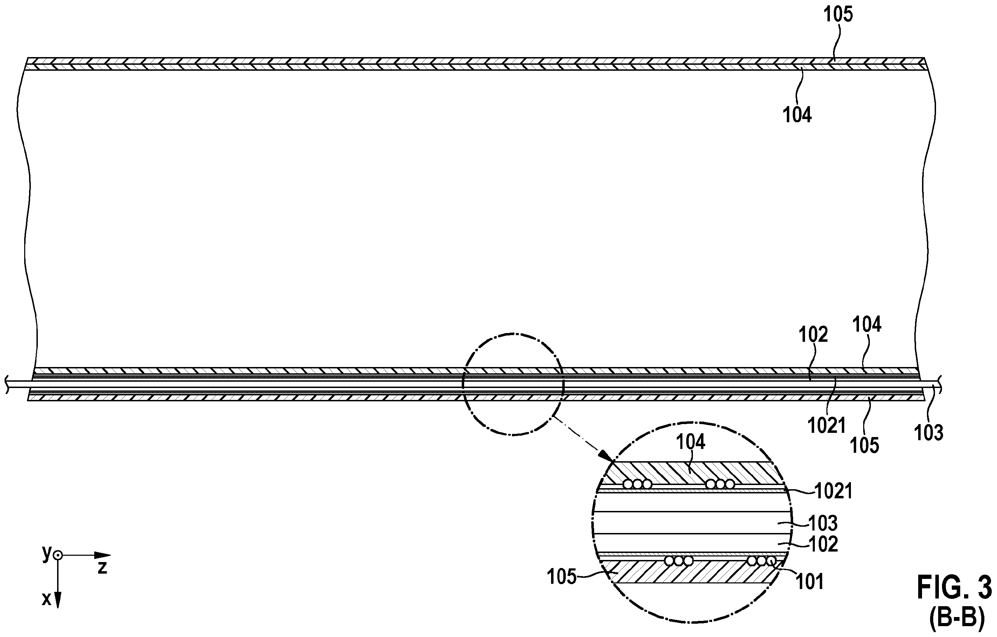

FIG. 3 schematically and by way of example shows a longitudinal section (along line A-A of FIG. 1) through the catheter tube 1. The cross-section B-B extends through a plane XZ parallel to the main extension direction Z of the catheter tube 1 of FIG. 1.

The mesh 101, in which, in turn, the guide lumen 102 is embedded, together with the pull element 103, as is shown in FIGS. 2 and 3, is arranged between the inner layer 104 and the outer layer 105. In the process, the guide lumen 102 is delimited by a guide lumen tube 1021, for example in the form of a Teflon tube, in the shown exemplary embodiment. In this way, it can be ensured that the pull element 103 is able to slide unimpaired within the tube wall 10 for transmitting a pulling force and is not, for example, blocked by the mesh 101 in the process.

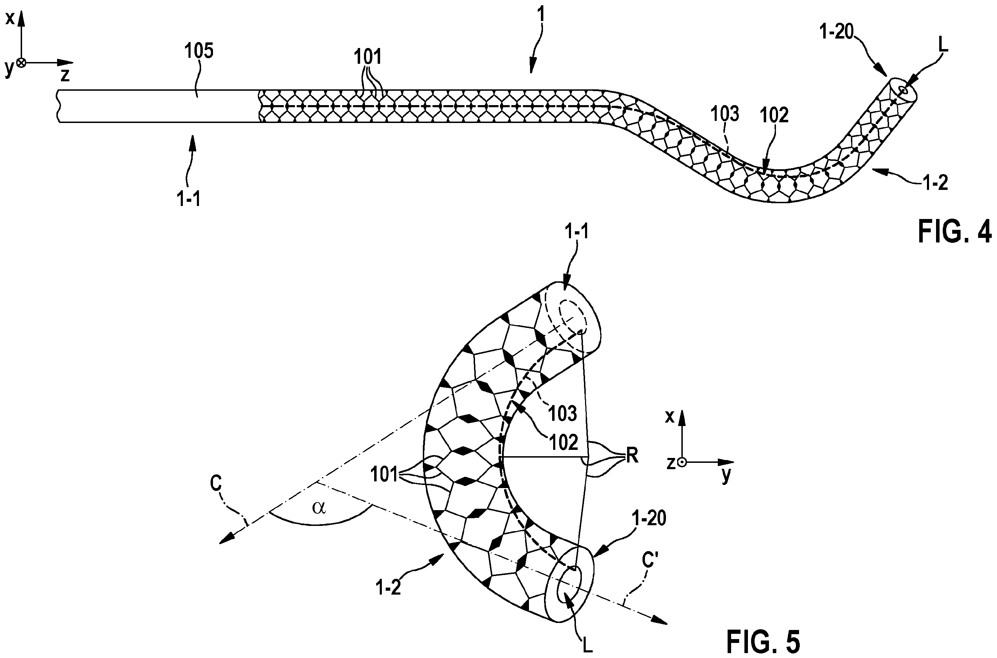

FIG. 4 represents the catheter tube of FIG. 1 in a formed state. For example, the catheter tube 1 can be transferred from the non-deformed state shown in FIG. 1 into the formed state shown in FIG. 4 by a pulling actuation of the pull element 103. As is shown by way of example and schematically in FIG. 4, the catheter tube 1 can be deformed in the process in such a way the distal portion 1-2 is bent with respect to the main extension axis Z of the proximal portion 1-1. In FIG. 4, the distal end 1-20 extends, for example with a considerable directional component, in the direction X that is perpendicular to the main extension plane Z.

At the same time, a partial deflection in the direction Y, which is perpendicular to directions X and Z, can take place. In this way, the distal end 1-20 of the catheter tube 1 can, for example, not only be "bent" in the manner of a straw (corresponding to a two-dimensional deformation, for example within a plane XZ through the main extension axis Z), but it is also possible, by an actuation of the pull element 103, to effectuate a three-dimensional deformation of the distal portion 1-2 of the catheter tube 1. As is illustrated in FIG. 4, the deformed distal portion 1-2 of the catheter tube 1 can, for example, follow at least a section of a helical path around the main extension plane Z.

For further illustration, FIG. 5 shows a shaped distal portion 1-2 of the catheter tube 1 in a projection along a main extension axis Z (that is, a projection into plane XY). It is apparent from this that the catheter tube 1 can be deformed by an actuation of the pull element 103 in such a way that, as a result, respective local extension axes C, C' of the distal portion 1-2, as seen in a projection along the main extension axis Z of the catheter tube 1 (in particular along a main extension axis Z of the proximal portion 1-1), pass over an angle α of at least 30°. However, as is shown by way of example in Fig. 5, the passed over angle α can also be larger than 90°. The angle α is preferably in the range of 30° to 270°.

Furthermore, as is likewise illustrated in FIG. 5, the formed distal portion 1-2, in the projection along the main extension axis Z, can at least approximately describe a section of a circular arc, wherein an associated radius R is preferably in the range of 2 mm to 100 mm.

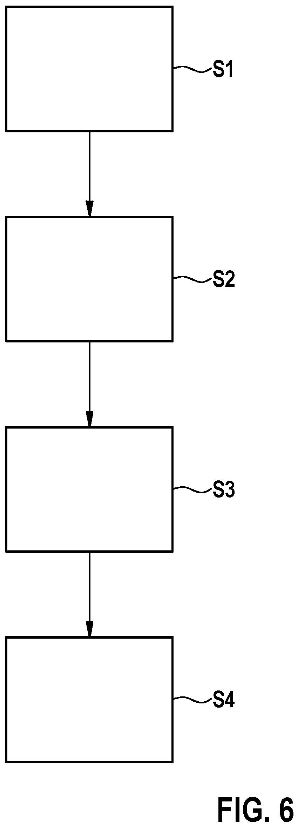

FIG. 6 shows a schematic block diagram including method steps of an exemplary method for implanting an implantable medical device by means of a steerable catheter comprising a catheter tube 1 according to the above-described type.

The method comprises the following steps:
In a first step S1, the catheter is inserted into the patient's body, and the distal portion 1-2 of the catheter tube 1 is advanced into the vicinity of a desired implantation site.

For example, the catheter tube 1 can be in the first, non-formed state shown in FIG. 1, which can also be referred to as a relaxed or neutral state, during insertion and advancement (step S). For example, it can be provided that no, or at the most a low, pulling force is exerted by means of the pull element 103 in the non-formed state.

In a second step S2, the distal portion 1-2 is deformed, by an actuation of the pull element 103, in such a way that the distal end 1-20 of the catheter tube 1 rests against the tissue. In the process, the distal end 1-20 assumes a second, formed state in which it can be more rigid, for example, than in the first state. In the second state, the distal end 1-20 of the catheter tube 1 can, for example, be braced with a relatively high force against the tissue.

A third step S3 provides a fixation of the implantable medical device at the desired implantation site by means of the catheter. In the process, the catheter tube 1 can, for example, remain in the second, formed state.

Finally, the catheter is removed from the patient's body in a fourth step S4. For removal, the catheter tube 1 can (by renewed actuation of the pull element 103, for example such that a pulling force is released) be brought into the first, non-formed state again. In this way, the retraction of the catheter tube 1 through the vessels of the patient can be facilitated, as a result of a greater flexibility of the distal portion 1-2.

The implantable medical device can, for example, be a cardiac pacemaker electrode lead, which is implanted in a location in the patient's heart suitable for HIS bundle pacing, using the described method. As an alternative, the implantable medical device can be a sensor, such as a pressure sensor, which is implanted into a pulmonary artery by means of the described method. Still another embodiment provides that the implantable medical device is an ILP, which is implanted into the heart.

Further possible details and optional additional method steps in connection with such methods that use a catheter tube 1 according to the invention were already described above.

It shall be mentioned that a catheter tube 1 according to the invention can also be employed in applications other than those described in greater detail above, such as for steering devices for cryo applications, as well as for targeted drug delivery, or for steering ablation or diagnostic catheter.

The scope of the present invention is defined by the appended claims.

### LIST OF REFERENCE NUMERALS

- 1: catheter tube
- 1-1: proximal portion
- 1-2: distal portion
- 1-20: distal end
- 10: tube wall
- 101: mesh
- 102: guide lumen
- 1021: guide lumen tube
- 103: pull element
- 104: inner layer
- 105: outer layer

- C, C': local extension axes
- L: tube lumen
- S1-S4: method steps

## Claims

1. A catheter tube (1) for a steerable catheter, comprising a tube wall (10) surrounding a tube lumen (L), the tube wall (10) comprising the following:
- a mesh (101); and
- a guide lumen (102) around which the mesh (101) is braided and in which a pull element (103) extends from a proximal portion (1-1) of the catheter tube (1) to a distal portion (1-2) of the catheter tube (1), wherein the pull element (103) is guided axially along the catheter tube (1),
the pull element (103) being connected in a tension-resistant manner to the tube wall (10) in the distal portion (1-2), and
the guide lumen (102) guiding the pull element (103) at least partially around the tube lumen (L), wherein the guide lumen (102) describes at least a section of a helical path around the tube lumen (L) in the distal portion.

2. The catheter tube (1) according to claim 1, **characterized in that** the guide lumen (102) extends across a circumferential angle of at least 30° in the tube wall.

3. The catheter tube (1) according to any one of the preceding claims, **characterized in that** the guide lumen (102) is delimited by a guide lumen tube (1021).

4. The catheter tube (1) according to claim 3, **characterized in that** the guide lumen tube (1021) comprises Teflon.

5. The catheter tube (1) according to any one of the preceding claims, **characterized in that** the distal portion (1-2) can be deformed three-dimensionally by an actuation of the pull element (103).

6. The catheter tube (1) according to claim 5, **characterized in that** the catheter tube (1) can be deformed, by an actuation of the pull element (103), in such a way that the distal portion (1-2) is bent with respect to a main extension axis (Z) of the proximal portion (1-1).

7. The catheter tube (1) according to claim 5 or 6, **characterized in that** the catheter tube (1) can be deformed, by an actuation of the pull element (103), in such a way that the distal portion (1-2) describes at least a section of a spiral-shaped or helical path.

8. The catheter tube (1) according to any one of claims 5 to 7, **characterized in that** the catheter tube (1) can be deformed, by an actuation of the pull element (103), in such a way that local extension axes (C, C') of the distal portion (1-2), as seen in a projection along a main extension axis (Z) of the catheter tube (1), pass over an angle (α) in the range of at least 30°.

9. The catheter tube (1) according to any one of claims 5 to 7, **characterized in that** the catheter tube (1) can be deformed, by an actuation of the pull element (103), in such a way that local extension axes (C, C') of the distal portion (1-2), in a projection along a main extension axis (Z) of the catheter tube (1), describe at least approximately a section of a circular arc having a radius (R) in the range of 2 mm to 100 mm.

10. The catheter tube (1) according to any one of the preceding claims, **characterized in that** the catheter tube (1) comprises a plurality of tube segments having differing rigidities.

## Patentansprüche

1. Katheterschlauch (1) für einen steuerbaren Katheter, mit einer Schlauchwand (10), die ein Schlauchlumen (L) umgibt, wobei die Schlauchwand (10) Folgendes umfasst:
- ein Netz (101); und
- ein Führungslumen (102), um das das Netz (101) geflochten ist und in dem sich ein Zugelement (103) von einem proximalen Abschnitt (1-1) des Katheterschlauchs (1) zu einem distalen Abschnitt (1-2) des Katheterschlauchs (1) erstreckt, wobei das Zugelement (103) axial entlang des Katheterschlauchs (1) geführt wird,
das Zugelement (103) im distalen Bereich (1-2) zugfest mit der Schlauchwand (10) verbunden ist, und
das Führungslumen (102) das Zugelement (103) zumindest teilweise um das Schlauchlumen (L) führt, wobei das Führungslumen (102) zumindest einen Abschnitt einer schraubenförmigen Bahn um das Schlauchlumen (L) im distalen Teil beschreibt.

2. Katheterschlauch (1) nach Anspruch 1, **dadurch gekennzeichnet, dass** sich das Führungslumen (102) über einen Umfangswinkel von mindestens 30° in der Schlauchwand erstreckt.

3. Katheterschlauch (1) nach einem der vorhergehenden Ansprüche, **dadurch gekennzeichnet, dass** das Führungslumen (102) durch ein Führungslumenschlauch (1021) begrenzt ist.

4. Katheterschlauch (1) nach Anspruch 3, **dadurch gekennzeichnet, dass** der Führungslumenschlauch (1021) aus Teflon besteht.

5. Katheterschlauch (1) nach einem der vorhergehenden Ansprüche, **dadurch gekennzeichnet, dass** der distale Abschnitt (1-2) durch eine Betätigung des Zugelements (103) dreidimensional verformbar ist.

6. Katheterschlauch (1) nach Anspruch 5, **dadurch gekennzeichnet, dass** der Katheterschlauch (1) durch eine Betätigung des Zugelements (103) derart verformbar ist, dass der distale Abschnitt (1-2) in Bezug auf eine Haupterstreckungsachse (Z) des proximalen Abschnitts (1-1) gebogen wird.

7. Katheterschlauch (1) nach Anspruch 5 oder 6, **dadurch gekennzeichnet, dass** der Katheterschlauch (1) durch eine Betätigung des Zugelements (103) derart verformbar ist, dass der distale Abschnitt (1-2) zumindest einen Abschnitt einer spiralförmigen oder schraubenförmigen Bahn beschreibt.

8. Katheterschlauch (1) nach einem der Ansprüche 5 bis 7, **dadurch gekennzeichnet, dass** der Katheterschlauch (1) durch eine Betätigung des Zugelements (103) derart verformbar ist, dass lokale Erstreckungsachsen (C, C') des distalen Abschnitts (1-2), gesehen in einer Projektion entlang einer Haupterstreckungsachse (Z) des Katheterschlauchs (1), einen Winkel (α) im Bereich von mindestens 30° überstreichen.

9. Katheterschlauch (1) nach einem der Ansprüche 5 bis 7, **dadurch gekennzeichnet, dass** der Katheterschlauch (1) durch eine Betätigung des Zugelements (103) so verformbar ist, dass lokale Erstreckungsachsen (C, C') des distalen Abschnitts (1-2) in einer Projektion entlang einer Haupterstreckungsachse (Z) des Katheterschlauchs (1) zumindest annähernd einen Kreisbogenabschnitt mit einem Radius (R) im Bereich von 2 mm bis 100 mm beschreiben.

10. Katheterschlauch (1) nach einem der vorhergehenden Ansprüche, **dadurch gekennzeichnet, dass** der Katheterschlauch (1) aus mehreren Schlauchsegmenten mit unterschiedlichen Steifigkeiten besteht.

## Revendications

1. Tuyau de cathéter (1) pour un cathéter dirigeable, comprenant une paroi de tuyau (10) entourant une lumière de tuyau (L), la paroi de tuyau (10) comprenant les éléments suivants :
- un maillage (101) ; et
- une lumière de guidage (102) autour de laquelle le maillage (101) est tressé et dans laquelle un élément de tirage (103) s'étend depuis une partie proximale (1-1) du tuyau de cathéter (1) jusqu'à une partie distale (1-2) du tuyau de cathéter (1), dans lequel l'élément de tirage (103) est guidé de manière axiale le long du tuyau de cathéter (1),
l'élément de tirage (103) étant connecté de manière résistante en tension à la paroi de tuyau (10) dans la partie distale (1-2), et
la lumière de guidage (102) guidant l'élément de tirage (103) au moins partiellement autour de la lumière de tuyau (L), dans lequel la lumière de guidage (102) décrit au moins une section d'un trajet hélicoïdal autour de la lumière de tuyau (L) dans la partie distale.

2. Tuyau de cathéter (1) selon la revendication 1, **caractérisé en ce que** la lumière de guidage (102) s'étend sur un angle circonférentiel d'au moins 30° dans la paroi de tuyau.

3. Tuyau de cathéter (1) selon l'une quelconque des revendications précédentes, **caractérisé en ce que** la lumière de guidage (102) est délimitée par un tuyau de lumière de guidage (1021).

4. Tuyau de cathéter (1) selon la revendication 3, **caractérisé en ce que** le tuyau de lumière de guidage (1021) comprend du téflon.

5. Tuyau de cathéter (1) selon l'une quelconque des revendications précédentes, **caractérisé en ce que** la partie distale (1-2) peut être déformée en trois dimensions par un actionnement de l'élément de tirage (103).

6. Tuyau de cathéter (1) selon la revendication 5, **caractérisé ce que** le tuyau de cathéter (1) peut être déformé, par un actionnement de l'élément de tirage (103), d'une façon telle que la partie distale (1-2) est pliée par rapport à un axe d'extension principal (Z) de la partie proximale (1-1).

7. Tuyau de cathéter (1) selon la revendication 5 ou 6, **caractérisé ce que** le tuyau de cathéter (1) peut être déformé, par un actionnement de l'élément de tirage (103), d'une façon telle que la partie distale (1-2) décrit au moins une section d'un trajet en spirale ou hélicoïdal.

8. Tuyau de cathéter (1) selon l'une quelconque des revendications 5 à 7, **caractérisé ce que** le tuyau de cathéter (1) peut être déformé, par un actionnement de l'élément de tirage (103), d'une façon telle que les axes d'extension locaux (C, C') de la partie distale (1-2), comme indiqué dans une projection le long d'un axe d'extension principal (Z) du tuyau de cathéter (1), passent sur un angle (α) dans la plage d'au moins 30°.

9. Tuyau de cathéter (1) selon l'une quelconque des revendications 5 à 7, **caractérisé ce que** le tuyau de cathéter (1) peut être déformé, par un actionnement de l'élément de tirage (103), d'une façon telle que les axes d'extension locaux (C, C') de la partie distale (1-2), dans une projection le long d'un axe d'extension principal (Z) du tuyau de cathéter (1), décrivent au moins approximativement une section d'un arc circulaire ayant un rayon (R) dans la plage de 2 mm à 100 mm.

10. Tuyau de cathéter (1) selon l'une quelconque des revendications précédentes, **caractérisé en ce que** le tuyau de cathéter (1) comprend une pluralité de segments de tuyau ayant des rigidités différentes.
